(19) Europäisches Patentamt
European Patent Office
Office européen des brevets

(11) Veröffentlichungsnummer: **0 212 373 B1**

(12) **EUROPÄISCHE PATENTSCHRIFT**

(45) Veröffentlichungstag der Patentschrift: **23.01.91 Patentblatt 91/04**

(51) Int. Cl.$^5$: **C07D 213/89,** C07D 405/12

(21) Anmeldenummer: **86110548.4**

(22) Anmeldetag: **30.07.86**

(54) **Photoreaktive Verbindungen.**

(30) Priorität: **03.08.85 DE 3527890**

(43) Veröffentlichungstag der Anmeldung: **04.03.87 Patentblatt 87/10**

(45) Bekanntmachung des Hinweises auf die Patenterteilung: **23.01.91 Patentblatt 91/04**

(84) Benannte Vertragsstaaten: **BE CH DE FR GB IT LI NL SE**

(56) Entgegenhaltungen:
**J. Streith: Pure & Appl. Chemistry, Vol. 49, S. 305-315, 1977**
**H.-J. Timpe: Heterocycl. Chem., Vol. 17, S. 213-253, 1974**
**T. Tsuchiya: J. Chem. Soc. Chem. Comm., S. 250-251, 1976**

(73) Patentinhaber: **BASF Aktiengesellschaft**
**Carl-Bosch-Strasse 38**
**D-6700 Ludwigshafen (DE)**

(72) Erfinder: **Koch, Horst, Dr.**
**Schliffgasse 22**
**D-6711 Grosskarlbach (DE)**
Erfinder: **Loerzer, Thomas, Dr.**
**Carl-Bosch-Ring 2**
**D-6710 Frankenthal (DE)**
Erfinder: **Boettcher, Andreas, Dr.**
**Schiesier Strasse 17**
**D-6704 Mutterstadt (DE)**
Erfinder: **Schulz, Guenther, Dr.**
**Wasgaustrasse 24**
**D-6700 Ludwigshafen (DE)**
Erfinder: **Schwalm, Reinhold, Dr.**
**Am Huettenwingert 53**
**D-6706 Wachenheim (DE)**

**EP 0 212 373 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft photoreaktive, niedermolekulare Verbindungen mit Iminopyridinium-ylid-Strukturen bzw. Iminopyrazinium-ylid-Strukturen, die unter dem Einfluß von aktinischem Licht photochemische Strukturänderungen eingehen.

Es ist bekannt, daß 1-Iminopyridinium-ylide, die am Pyridin-Stickstoffatom beispielsweise Alkoxycarbonylimino-, Benzoylimino-oder Tosylimino-Gruppen enthalten, unter der Einwirkung von aktinischem Licht einer Wellenlänge von etwa 320 nm Strukturänderungen, z.B. zu Diazepinen, erfahren. Der Pyridinrest kann dabei durch Elektronendonator-Gruppen substituiert sein, wodurch die photoinduzierte Ringerweiterung begünstigt wird. Beispiele solcher Elektronendonor-Substituenten sind die Methyl-, Phenyl- und Dimethylamino-Gruppe oder auch Halogenatome. Eine ausführliche Abhandlung der Photochemie der 1-Iminopyridinium-ylide findet sich beispielsweise bei J. Streith, Pure and Applied Chemistry, Vol. 49 (1977), Seiten 305 bis 315, und H.-J. Timpe, Adv. Heterocycl. Chem., Vol. 17 (1974), Seiten 213 bis 253. Eine ähnliche Photoreaktion gehen analoge 1-Iminopyrazinium-ylide ein, die unter der Einwirkung von aktinischem Licht über Triazepin-Zwischenstufen letztlich Pyrazol-Derivate bilden (vgl. T. Tsuchiya, J.Chem.Soc.Chem. Comm. (1976), Seiten 250 bis 251). Die photoinitiierte Umlagerung der Iminopyridinium-ylide eröffnet eine Synthesemöglichkeit für die Diazepine.

In vielen Gebieten der Technik werden Systeme gefordert, die unter Einwirkung von aktinischer Strahlung ihre Eigenschaften ändern. Hierzu gehören beispielsweise härtbare Überzüge, Schutzschichten oder Anstriche. Weitere Beispiele sind die Systeme für die optische Informationsfixierung, wie beispielsweise zur Herstellung von Hochdruckplatten, Tiefdruckplatten, Flachdruckplatten oder Resistmustern für die Halbleitertechnik und ähnliche. Gleichermaßen können solche Systeme bei in vivo-Systemen als photoreaktive Agentien z.B. zur Membranveränderung eingesetzt werden. In den genannten Anwendungsbereichen spielt im allgemeinen die Änderung der mechanischen und sonstigen physikalischen Eigenschaften eine wesentliche Rolle. Die gewünschten Eigenschaftsänderungen werden in den hierfür bekannten photoempfindlichen Systemen im allgemeinen über Photopolymerisations- bzw. Photovernetzungs-Reaktionen oder über Photoabbau-Reaktionen erzielt.

Aufgabe der vorliegenden Erfindung war es, neue, photoreaktive Verbindungen mit vorteilhaften Eigenschaften, insbesondere hoher Photoreaktivität und einer guten Photoempfindlichkeit über einen breiten spektralen Wel- lenlängenbereich, zur Verfügung zu stellen, deren Eigenschaftsbild in großem Umfang geändert werden kann und die dadurch breite und vielfältige Anwendungsmöglichkeiten besitzen. Insbesondere sollen sich die neuen photoreaktiven Verbindungen mit Vorteil für den Einsatz bei der Herstellung von Überzügen oder Schutzschichten sowie in der optischen Informationsfixierung eignen.

Es wurde nun gefunden, daß diese Aufgabe durch die speziellen, nachfolgend näher gekennzeichneten 1-Iminopyridinium-ylide bzw. 1-Iminopyrazinium-ylide gelöst wird, die im Pyridin- bzw. Pyrazin-Ring durch mindestens eine aliphatisch ungesättigte Gruppe substituiert sind.

Gegenstand der Erfindung sind demzufolge niedermolekulare, photoreaktive Verbindungen der allgemeinen Formel (I) bzw. (II)

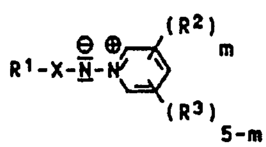

(I)

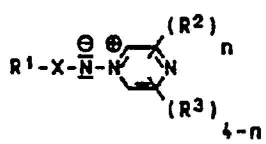

(II)

wobei bedeuten

X: 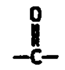 , 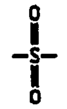 , 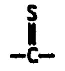 , 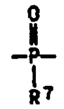 ,

$R^1$ : ein Phenylrest, ein durch Halogen, Alkyl-, Alkoxy-, Amino- oder (Meth)acryloylreste tragende Gruppen substituierter Phenylrest, ein Styrylrest, ein Alkylrest, ein substituierter Alkenylrest, ein vinylisch ungesättigter aliphatischer Rest sowie für den Fall

X= 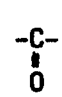 oder 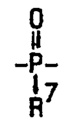

auch eine $-OR^4$-Gruppe oder eine $-NR^5R^6$-Gruppe mit den weiter unter für $R^4$, $R^5$, $R^6$ und $R^7$ angegebenen Bedeutungen ; $R^2$ : ein Rest mit einer Vinyl-Gruppe, einer Alkenyl-Gruppe, einer Alkinyl-Gruppe, einer Alk-dien-yl-Gruppe, einer Alk-diin-yl-Gruppe, einer Acryloyl-Gruppe, einer Methacryloyl-Gruppe oder einer Fumarsäure- oder Maleinsäure-Gruppe ;

$R^3$ : ein Wasserstoffatom, eine, gegebenenfalls substituierte, Alkyl-Gruppe, eine, gegebenenfalls substituierte, Phenyl-Gruppe, ein Halogenatom, eine Amino-Gruppe, ein Alkoxy-Rest, ein Phenoxy-Rest, eine Nitro-Gruppe, eine Cyano-Gruppe, ein Alkoxycarbonyl-Rest bzw. ein Aminocarbonyl-Rest oder ein anelliertes alicyclisches, aromatisches oder heteroaromatisches Ringsystem, beispielsweise ein benzoanelliertes Ringsystem ;

$R^4$, $R^6$ : Alkyl, Alkenyl, Alkinyl, Alk-dien-yl, Alk-diin-yl, ein eine Acryloyl-Gruppe oder Methacryloyl-Gruppe enthaltender Rest, Hydroxyalkyl, ein Oligoalkylenoxid-Rest oder der Styrol-Rest ;

$R^5$ : ein Wasserstoffatom, ein Alkyl-Rest, Cycloalkyl-Rest, der Phenyl-Rest oder ein substituierter Alkyl- oder Phenyl-Rest ;

$R^7$ : ein gegebenenfalls substituierter Alkyl-, Alkoxy- oder Aryl-Rest, ein Halogenatom oder eine Hydroxyl-Gruppe ;

m, n : eine ganze Zahl von 1 bis 3.

Die erfindungsgemäßen, photoreaktiven Verbindungen sind hydrophil, haben eine hohe Photoreaktivität und besitzen, in Abhängigkeit von den Substituenten, eine große Photoempfindlichkeit über einen breiten spektralen Wellenlängenbereich. Somit ist es möglich, die Photoreaktion an der Ylid-Gruppe, die zu einer chemischen Strukturänderung führt, mit aktinischer Strahlung einer Wellenlänge zu induzieren, die außerhalb der Eigenabsorption der Ylid-Gruppierung liegt. Durch den Gehalt an aliphatischen Mehrfachbindungen in den erfindungsgemäßen Verbindungen zusätzlich zu den photoreaktiven Ylid-Gruppen ist es in einfacher und sehr vorteilhafter Weise möglich, die Eigenschaften der erfindungsgemäßen Verbindungen in starkem Ausmaß und sehr breitem Umfang zu ändern, wobei die durch die Photoreaktionen der Ylid-Gruppierungen bewirkten Eigenschaftsänderungen durch vorherige, gleichzeitige oder nachträgliche Umsetzung der im Molekül enthaltenen aliphatischen Mehrfachbindungen beispielsweise verstärkt und/oder modifiziert werden können. Die erfindungsgemäßen photoreaktiven Verbindungen können in starkem Ausmaß hydrophobiert werden. Gleichzeitig läßt sich auch das Adhäsionsvermögen zu Substraten und gebräuchlichen Trägermaterialien in hohem Maße verändern. Ferner lassen sich die Lösungseigenschaften in den verschiedenen Lösungsmitteln oder Lösungsmittelgemischen variieren und somit z.b. Produkte herstellen, die im Vergleich zu den Ausgangsverbindungen eine hohe mechanische Beständigkeit und Resistenz gegenüber polaren Lösungsmitteln aufweisen. Auch kann beispielsweise die Permeabilität der erfindungsgemäßen photoreaktiven Verbindungen durch Membrane einfach und in großem Umfang geändert werden, was für deren Anwendung in biologischen Systemen von Bedeutung ist. Über die aliphatischen Mehrfachbindungen können mit den erfindungsgemäßen Verbindungen insbesondere Anlagerungs-, Polymerisations- oder Vernetzungsreaktionen durchgeführt werden. Die erfindungsgemäßen Verbindungen eignen sich mit Vorteil für solche Anwendungen, in denen es auf eine große Änderung in den hydrophilen und hydrophoben Eigenschaften, auf starke Haftungsdifferenzierung und hohe Beständigkeit der erhaltenen Produkte ankommt.

Bei den erfindungsgemäßen photoreaktiven Verbindungen handelt es sich um 1-Iminopyridinium-ylide bzw. 1-Iminopyrazinium-ylide, bei denen der Pyridin- bzw. Pyrazin-Rest durch mindestens eine ungesättigte Gruppe mit mindestens einer aliphatischen Mehrfachbindung substituiert ist. In den oben angegebenen allgemeinen Formeln (I) und (II) ist m bzw. n vorzugsweise 1 oder 2, X vorzugsweise die Carbonylgruppe und

haben die Reste $R^1$ bis $R^3$ insbesondere folgende Bedeutung :

$R^1$ : Phenyl, ein substituierter Phenylrest, beispielsweise ein durch Halogen, Alkyl-, Alkoxy-, Amino- oder (Meth)acryloylreste tragende Gruppen substituierter Phenylrest oder der Styryl-Rest, ein Alkylrest, ein substituierter Alkenylrest, ein vinylisch ungesättigter aliphatischer Rest sowie - für den Fall

X = 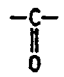 oder 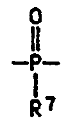

(mit der vorstehend für $R^7$ angegebenen Bedeutung) - auch eine $-OR^4$ Gruppe oder eine $-NR^5R^6$-Gruppe mit den weiter unten für $R^4$, $R^5$ und $R^6$ angegebenen bevorzugten Bedeutungen ;

$R^2$ : ein Rest mit einer Vinyl-Gruppe, einer Alkenyl-Gruppe, einer Alkinyl-Gruppe, einer Alk-dien-yl-Gruppe, einer Alk-diin-yl-Gruppe, einer Acryloyl-Gruppe, einer Methacryloyl-Gruppe oder einer Fumarsäure-oder Maleinsäure-Gruppe ;

bevorzugt ist, daß in den Formeln (I) bzw. (II) $R^2$ einen Rest mit einer oder mehreren aliphatischen Doppelbindungen darstellt oder $R^2$ ein Rest mit einer oder mehreren acetylenischen Dreifachbindungen ist. Besonders bevorzugt ist, daß in den allgemeinen Formeln (I) bzw. (II) der Rest $R^2$ mehrere, gegebenenfalls miteinander in Konjugation stehende, aliphatische Mehrfachbindungen aufweist, bzw. daß in den Formeln (I) bzw. (II) die aliphatischen Mehrfachbindungen in dem Rest $R^2$ in Konjugation zu den Mehrfachbindungen des Pyridinium- bzw. Pyrazinium-Ringes stehen.

$R^3$ : ein Wasserstoffatom, eine gegebenenfalls substituierte Alkyl-Gruppe, eine gegebenenfalls substituierte Phenyl-Gruppe, ein Halogenatom, eine Amino-Gruppe, ein Alkoxy-Rest, ein Phenoxy-Rest, eine Nitro-Gruppe, eine Cyano-Gruppe, ein Alkoxycarbonyl-Rest bzw. ein Aminocarbonyl-Rest oder ein anelliertes alicyclisches, aromatisches oder heteroaromatisches Ringsystem, beispielsweise ein benzoanelliertes Ringsystem ;

$R^4$, $R^6$ : Alkyl, Alkenyl, Alkinyl, Alk-dien-yl, Alk-diin-yl, ein eine Acryloyl-Gruppe oder Methacryloyl-Gruppe enthaltender Rest, Hydroxyalkyl, ein Oligoalkylenoxid-Rest oder der Styrol-Rest ;

$R^5$ : ein Wasserstoffatom, ein Alkylrest, Cycloalkylrest, der Phenylrest oder ein substituierter Alkyl- oder Phenylrest.

In einer besonderen Ausführungsform können dabei die erfindungsgemäßen Verbindungen der allgemeinen Formeln (I) und (II) auch zwei oder mehr Pyridinium-ylid- bzw. Pyrazinium-ylid-Gruppierungen enthalten. So kann beispielsweise $R^1$ ein aliphatischer oder aromatischer Rest, insbesondere ein Alkyl-, Alkenyl- oder Phenyl-Rest, sein, der durch eine Gruppe der Formel (III) oder (IV)

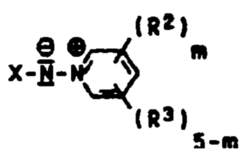 (III)

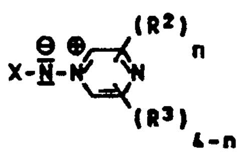 (IV)

substituiert ist, wobei $R^2$, $R^3$, X, m und n die vorstehend angegebenen Bedeutungen besitzen. Dabei trägt der Rest $R^1$ bei den Verbindungen der allgemeinen Formel (I) insbesondere eine Gruppe der Formel (III) und bei Verbindungen der allgemeinen Formel (II) insbesondere eine Gruppe der Formel (IV). Ebenso ist es möglich, daß bei den erfindungsgemäßen Verbindungen der allgemeinen Formeln (I) und (II) der Rest $R^3$ eine weitere Iminopyridinium- oder Iminopyrazinium-Ylid-Gruppierung enthält. So kann $R^3$ ein aliphatischer oder aromatischer Rest oder ein anelliertes Ringsystem sein, welcher bzw. welches eine Gruppierung der allgemeinen Formeln (V) oder (VI)

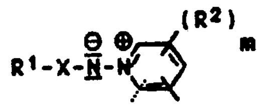

(V)

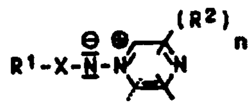

(VI)

enthält, worin $R^1$, $R^2$, X, m und n die oben angegebene Bedeutung haben. Hierbei enthält der Rest $R^3$ bei den Verbindungen der allgemeinen Formel (I) insbesondere eine Gruppierung der Formel (V) und bei den Verbindungen der Formel (II) insbesondere eine Gruppierung der Formel (VI). Bei den erfindungsgemäßen Verbindungen der allgemeinen Formeln (I) oder (II), die mehr als eine Iminopyridinium- bzw. Iminopyrazinium-Ylid-Gruppierung enthalten, ist diese üblicherweise entweder in $R^1$ oder in $R^3$ enthalten.

Durch geeignete Auswahl der Reste $R^1$, $R^2$, $R^3$ und X in den allgemeinen Formeln (I) und (II) können die Eigenschaften der erfindungsgemäßen photoreaktiven Verbindungen, wie beispielsweise die Photoempfindlichkeit, sehr stark variiert und beispielsweise für den gewünschten Anwendungszweck eingestellt werden. So ist es durch geeignete Wahl dieser Reste beispielsweise möglich, amphiphile Verbindungen zu erhalten. Nachfolgend werden die erfindungsgemäßen photoreaktiven Verbindungen beispielhaft näher beschrieben.

Beispiele für den Rest $R^1$ in den allgemeinen Formeln (I) und (II) sind : Phenyl, p-Methylphenyl, p-Chlorphenyl, o-Chlorphenyl, p-Bromphenyl, p-Aminophenyl, p-Diphenyl, p-Hydroxyphenyl, p-Methoxyphenyl,

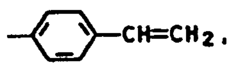, 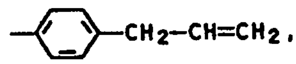, 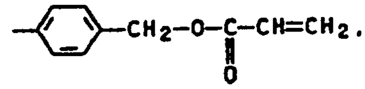,

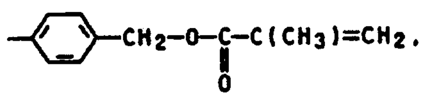, 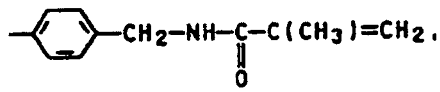,

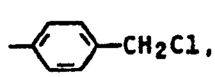, 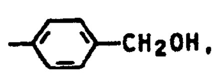, 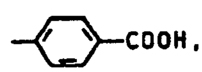, 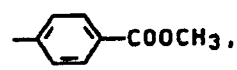,

Methyl, Ethyl, Trifluormethyl, 2-Aminoethyl, $-(CH_2)_4-SH$,

$-CH=CH_2$ oder $-CH_2-CH=CH_2$, aber auch 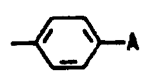, $-CH=CHA$

oder $-(CH_2)_4-A$

mit A gleich einem Rest der vorstehend angegebenen Formel (III) oder (IV). Für den Fall, daß X in den allgemeinen Formeln (I) oder (II) der

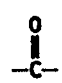 oder 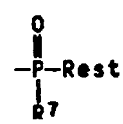

ist, kann $R^1$ auch eine $OR^4$- oder eine $NR^5R^6$-Gruppe sein, wobei illustrierende Beispiele für $R^4$, $R^5$ und $R^6$ weiter unten angeführt sind.

Beispiele für den Rest $R^2$ in den allgemeinen Formeln (I) bzw. (II) sind :

$-CH=CH_2$, $-CH_2-CH=CH_2$, 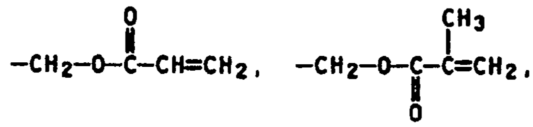

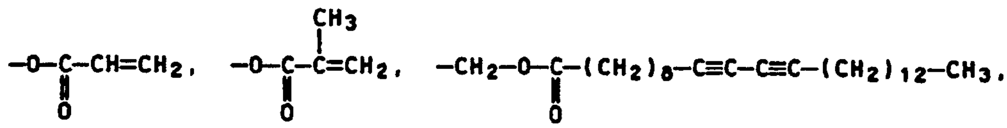

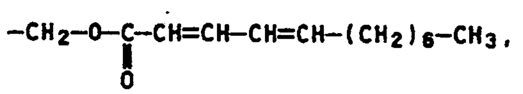 $-O-(CH_2)_{10}-CH=CH_2$,

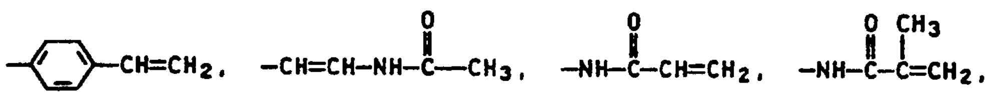

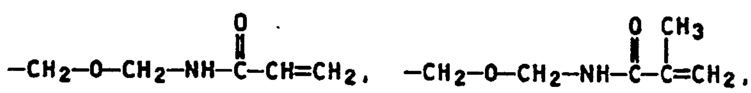

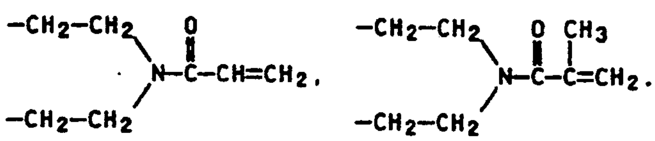

Beispiele für den Rest $R^3$ in den allgemeinen Formeln (I) bzw. (II) sind unter anderem das Wasserstoffatom, Methyl, n-Butyl, Phenyl, Methoxy, Phenoxy,

$-Cl$, $-NH_2$, $-NHCH_3$, $-NO_2$, $-C{\equiv}N$, 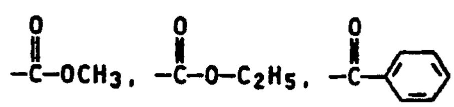

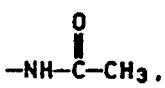 $-COOH$, $-CH_2-Cl$, $-CH_2-CH_2-OH$, $-CH_2-CH_2-SH$,

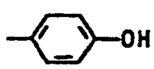 oder 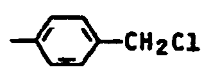

oder ringbildende Reste bzw. anellierte Systeme wie

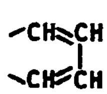, 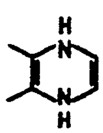, 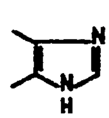, 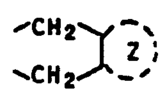 oder 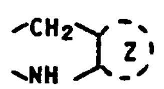,

worin Z für ein aromatisches oder heteroaromatisches Ringsystem steht, welches auch eine Gruppierung der vorstehend angegebenen allgemeinen Formeln (V) oder (VI) enthalten kann. $R^3$ kann ferner $-(CH_3)_p$-B sein, wobei p eine Zahl von 1 bis 10 und B eine Gruppierung der allgemeinen Formel (V) oder (VI) bedeuten.

Beispiele für die Reste $R^4$ und $R^6$ sind : Methyl, Ethyl, Propyl, Butyl, Phenyl, Allyl, 2-Hydroxyethyl, Tolyl, Xylyl, Aminophenyl, Styryl,

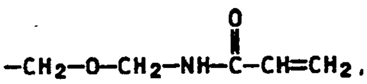,
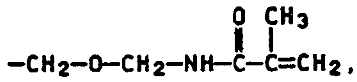,

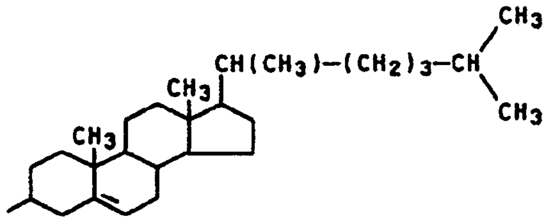

oder

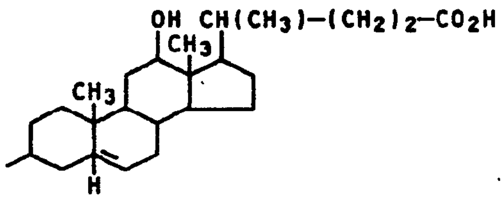

Als Beispiele für den Rest $R^5$ seien Methyl, Ethyl und Phenyl genannt.

Beispiele für erfindungsgemäße photoreaktive Verbindungen sind unter anderem die nachfolgend aufgeführten Substanzen :

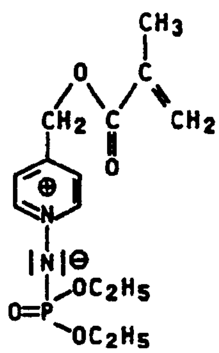

(VII)

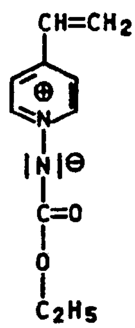

(VIII)

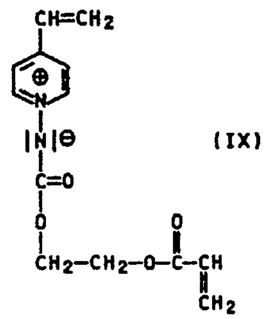

(IX)

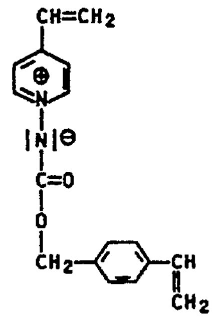

(X)

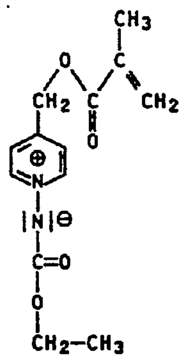

(XI)

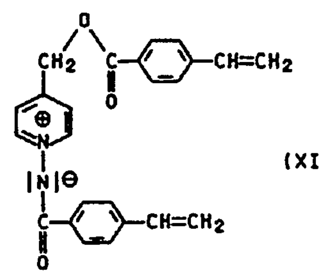

(XII)

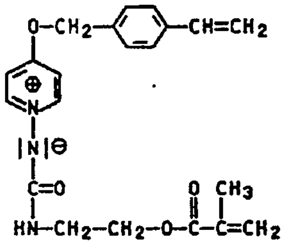

(XIII)

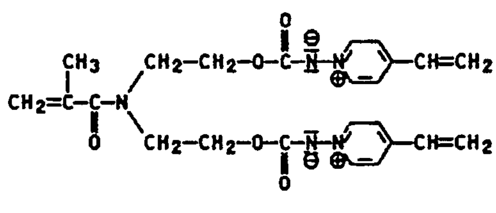

(XIV)

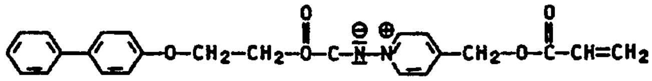

(XV)

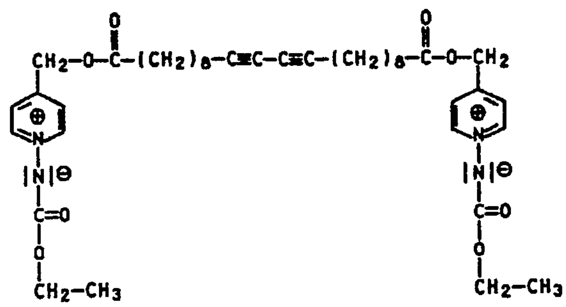

(XVI)

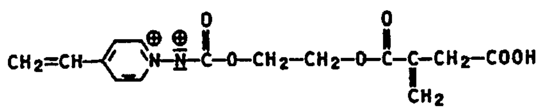

(XVII)

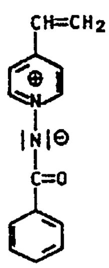

(XVIII)

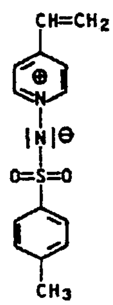

(XIX)

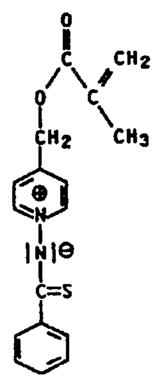

(XX)

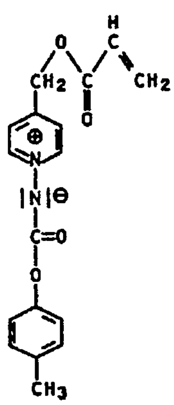

(XXI)

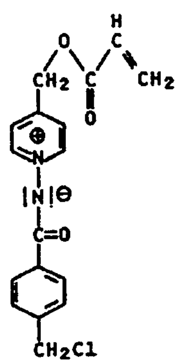

(XXII)

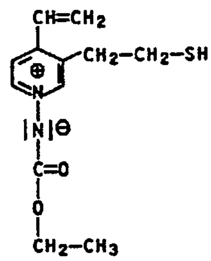

(XXIII)

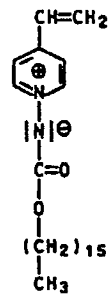

(XXIV)

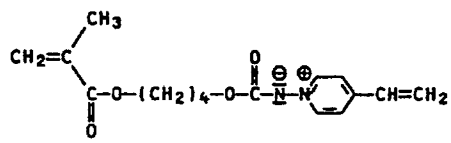

(XXV)

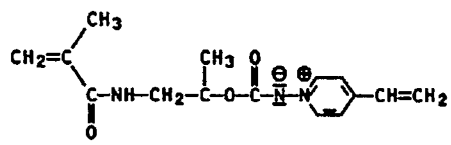

(XXVI)

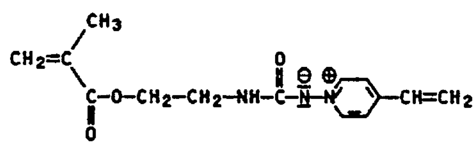

(XXVII)

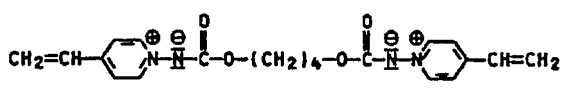

(XXVIII)

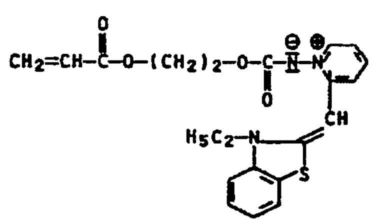

(XXIX)

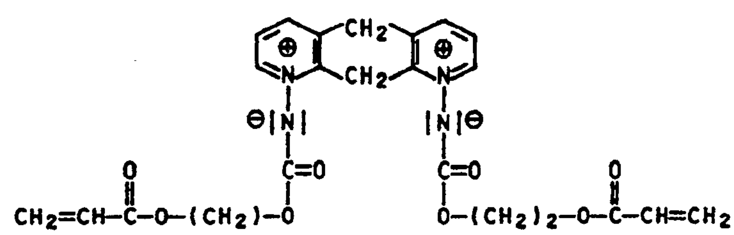

(XXX)

Bevorzugte Verbindungen hierunter sind die der Formeln (IX), (XI), (XIV), (XVII), (XXII), (XXIV), (XXVII) und (XXVIII).

Die erfindungsgemäßen photopolymerisierbaren Verbindungen können nach gängigen und an sich bekannten Methoden der organischen Chemie hergestellt werden. Hierbei werden die entsprechend substituierten Pyridine bzw. Pyrazine zunächst N-aminiert und anschließend zu den Yliden umgesetzt. Die N-Aminierung der substituierten Pyridine bzw. Pyrazine kann nach bekannten Verfahren beispielsweise in wäßrig-alkalischen Medien mit Hydroxylamin-O-sulfonsäure oder unter neutralen Bedingungen in organischen Lösungsmitteln mit o-Mesitylensulfonylhydroxylamin oder strukturverwandten Derivaten durchgeführt werden (vgl. R.G. Wallace, Org. Prep. Proc. Int. 14, 265 (1984) und H. Fritz et al, Helv. Chim. Acta 61, 2887 (1978)).

Die Umsetzung der substituierten Pyridine bzw. Pyrazine kann auch entsprechend J. Org. Chem. 35, 433 (1970) mit Azidoformiaten erfolgen. Die entstehenden Pyridinium- bzw. Pyraziniumverbindungen werden zur Herstellung der N-Iminopyridinium-ylide bzw. N-Iminopyrazinium-ylide unter wasserfreien Bedingungen in polaren Lösungsmitteln, wie z.B. Dimethylformamid oder Tetrahydrofuran, mit äquimolaren Mengen einer anorganischen Base, z.B. Kaliumcarbonat, oder einem basischen Ionenaustauscher umgesetzt. Zur Herstellung der gewünschten erfindungsgemäßen Verbindungen können die erhaltenen Ylide nach an sich bekannten Verfahren beispielsweise mit Ketenen, Isocyanaten, Sulfonylisocyanaten oder aktivierten Kohlesäureestern, wie Kohlesäureesteramiden, vorzugsweise durch Reaktion bei tiefen Temperaturen, umgesetzt werden. Die hierzu einzusetzenden Ketene, Isocyanate, Sulfonylisocyanate oder aktivierten Kohlensäureester können ebenfalls nach den für die Herstellung entsprechender Verbindungen gängigen Verfahren erhalten werden. Für die Herstellung bifunktioneller oder amphiphiler erfindungsgemäßer Ylid-Verbindungen sind auch solche Verfahrensweisen von Vorteil, bei denen z.B. in Analogie zu den Vorschriften von Sasaki et al., J. Org. Chem. 35, 426 (1970) die entsprechend substituierten Pyridine bzw. Pyrazine mit Hydroxylamino-O-sulfonsäure N-aminiert werden und das Rohprodukt z.B. in Alkohol oder Chloroform in Gegenwart von überschüssigem Kaliumcarbonat mit dem Kohlesäureesterchlorid umgesetzt wird.

Die erfindungsgemäßen 1-Imino-pyridinium-ylide bzw. 1-Imino-pyrazinium-ylide sind photoreaktiv und erfahren bei Bestrahlung mit aktinischem Licht eine chemische Strukturänderung. Die Pyridinium-ylide gehen hierbei je nach Strukturtyp und Substitutionsmuster in Diazepin-Derivate, in substituierte Aminopyridine oder in reaktive Fragmente über, wohingegen sich die Pyrazinium-ylide in Pyrazo-Derivate und reaktive Fragmente umwandeln. Durch diese photoinduzierte chemische Strukturänderung wird auch eine Änderung in den Eigenschaften dieser Verbindungen bewirkt. Insbesondere geht der ursprünglich hydrophile Charakter verloren und werden diese Verbindungen hydrophob. Da die erfindungsgemäßen niedermolekularen Pyridinium- bzw. Pyrazinium-Verbindungen neben der Imino-ylid-Struktur durch Substitution des Pyridin-bzw. Pyrazin-Rings noch mindestens einen Rest mit ein oder mehreren aliphatischen Doppel- und/oder Dreifachbindungen enthalten, lassen sich mit den erfindungsgemäßen Verbindungen eine Vielzahl von Produkten mit sehr vorteilhaften Eigenschaften und einem breiten Anwendungsbereich herstellen. Da sich erfindungsgemäß Imino-pyridinium-ylide bzw. Imino-pyrazinium-ylide mit einer sehr breiten spektralen Empfindlichkeit herstellen lassen, ist man für die photochemische Reaktion nicht auf aktinisches Licht einer Wellenlänge beschränkt, die der Eigenabsorption der Imino-ylid-Struktur entspricht, sondern kann zur Induzierung der photochemischen Strukturänderung dieser Verbindungen aktinisches Licht eines breiten Wellenlängenbereichs verwenden.

Im Vergleich zu den bekannten Imino-pyridinium-yliden bzw. Imino-pyrazinium-yliden erhält man mit den erfindungsgemäßen Verbindungen eine erheblich stärkere Differenzierung zwischen hydrophilen und hydrophoben Eigenschaften vor und nach der photoinduzierten Strukturumwandlung. Die erfindungsgemäßen Verbindungen sind dort von besonderem Vorteil, wo es auf eine Änderung der Grenzflächenaktivität bei der Anwendung ankommt. Auch besitzen die erfindungsgemäßen Verbindungen im photoreaktiven Zustand bzw. nach der photochemischen Strukturänderung stark unterschiedliche Haftungseigenschaften gegenüber Substraten, wie z.B. Metallen oder Kunststoff-Folien, so daß in mit den erfindungsgemäßen Verbindungen hergestellten Schichten durch bildmäßige Bestrahlung mit aktinischem Licht eine große photochemische Haftungsdifferenzierung erzielt werden kann. Die in den erfindungsgemäßen Verbindungen enthaltenen Substituenten mit mindestens einer aliphatischen Doppel- oder Dreifachbindung können dabei zur gezielten Einstellung gewünschter Eigenschaften ausgenutzt werden kann. Insbesondere eignen sich diese aliphatischen Doppel- oder Dreifachbindungen zur Änderung der mechanischen und physikalischen Eigenschaften von mit diesen Verbindungen hergestellten Schichten durch Polymerisation und Vernetzung der erfindungsgemäßen Verbindungen. Die aliphatischen Mehrfachbindungen stehen auch für weitere Umsetzungen, beispielsweise mit Persäuren zu Epoxiden oder mit Mercaptanen zu Thioethern zur Verfügung. Derartige Reaktionen können unter anderem in in vivo Systemen ablaufen.

Die erfindungsgemäßen niedermolekularen 1-Iminopyridinium-ylide bzw. 1-Iminopyrazinium-ylide eignen sich grundsätzlich für alle Anwendungen, bei denen auf photochemischem Weg eine Eigenschaftsänderung erzielt werden soll. Insbesondere vorteilhaft sind sie in den Anwendungsbereichen, in denen es auf eine Differenzierung zwischen Hydrophilie und Hydrophobie ankommt oder auch bei grenzflächenaktiven Systemen. Für die photochemische Strukturänderung der erfindungsgemäßen photoreaktiven Verbindungen kommt aktinisches Licht im Wellenlängenbereich von 220 bis 500 nm in Betracht. Als Lichtquellen können dabei die üblichen und an sich bekannten Lichtquellen für aktinisches Licht in diesem Wellenlängenbereich dienen. Anwendungsbeispiele für die erfindungsgemäßen niedermolekularen photoreaktiven Verbindungen sind z.B. die Naßfestausrüstung von photopolymeren Hochdruckplatten, der Einsatz als Schutzkolloid oder Emulgator in Dispersionen, bei denen nach der Anwendung der disperse Zustand aufgelöst werden soll, Pflanzenschutz, photohärtbare Lack-, Anstrich- und Reprographie-Systeme, die beispielsweise aus polaren, insbesondere wäßrigen, Lösungsmitteln oder Lösungsmittelgemischen verarbeitbar sind, nach der Härtung jedoch hydrophobe Eigenschaften aufweisen.

Die Erfindung wird durch die nachfolgenden Beispiele näher erläutert. Die in den Beispielen genannten Teile und Prozente beziehen sich, sofern nicht anders angegeben, auf das Gewicht.

## Beispiel 1

a) Herstellung von N-Amino-4-vinylpyridinium-mesitylsulfonat

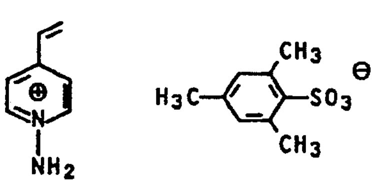

Zu einer gerührten Lösung von 52,5 Teilen 4-Vinylpyridin in 1000 Teilen Dichlormethan wurden 107,5 Teile Mesitylsulfonylhydroxylamin in 500 Teilen Dichlormethan bei 0°C zugetropft. Nach 2stündigem Rühren wurde das N-Amino-4-vinylpyridinium-mesitylsulfonat durch Zugabe von 1500 Teilen Ether ausgefällt und aus Essigester/Methanol umkristallisiert. Es wurden 118 Teile erhalten, die sofort weiter umgesetzt wurden. Ausbeute : 74 % der Theorie.

b) Herstellung von

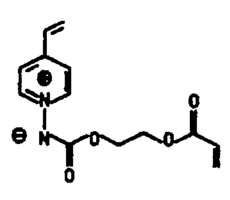

Eine Lösung von 64 Teilen N-Amino-4-vinylpyridinium-mesitylsulfonat in 1200 Teilen Tetrahydrofuran wurde mit 140 Teilen fein pulverisiertem Kaliumcarbonat versetzt, wobei eine violett-blaue Färbung entstand. 39,3 Teile Ethylenglycolmonochlorformiatmonoacrylat in 100 Teilen Tetrahydrofuran wurden dann so zugetropft, daß die blaue Färbung im Reaktionskolben erhalten blieb. Nach beendeter Zugabe wurde noch 2 Stunden gerührt. Die anorganischen Salze wurden abfiltriert. Die so erhaltene organische Phase wurde eingeengt, in Dichlormethan aufgenommen und mit wenig Natriumcarbonatlösung gewaschen. Nach dem Trocknen wurde das Lösungsmittel im Vakuum abgezogen und man erhielt 33,5 Teile

des Pyridinium-Ylids. Ausbeute : 64 % der Theorie. Die Substanz ist im Dünnschichtchromatogramm einheitlich.

Beispiele 2 bis 4

In analoger Weise wie in Beispiel 1 wurden die nachfolgenden Verbindungen hergestellt. Alle Verbindungen wurden durch Elementaranalyse, IR- und ¹H-NMR-Spektrum analysiert.

| Beispiel | Verbindung | Ausbeute |
|---|---|---|
| 2 | 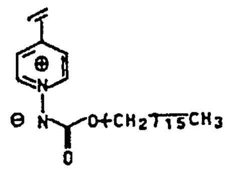 | 68 % |
| 3 | 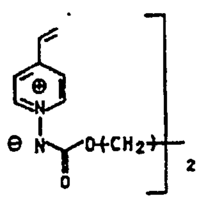 | 49 % |
| 4 | 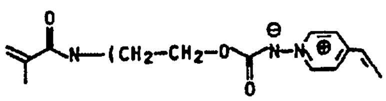 | 45 % |

Beispiel 5

Herstellung von

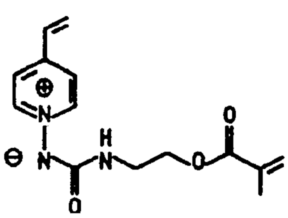

Eine Lösung von 44,4 Teilen N-Amino-4-vinyl-pyridiniumiodid in 1200 Teilen Tetrahydrofuran wurde mit 56 Teilen fein pulverisiertem Kaliumcarbonat versetzt, wobei eine violette Färbung entstand. Bei 0°C wurden 34 Teile Isocyanatoethylmethacrylat, in 100 Teilen Tetrahydrofuran gelöst, langsam zugetropft. Nach beendeter Zugabe wurde noch 2 Stunden lang bei Raumtemperatur gerührt und wie in Beispiel 1 aufgearbeitet. Die Ausbeute lag bei 69 % der Theorie. Die Charakterisierung der Verbindung erfolgte spektroskopisch und über Elementaranalyse.

Beispiel 6

a) Herstellung von 4-(Tetrahydropyran-2-yloxy)-methylpyridin (1)

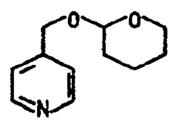

Durch eine Lösung, bestehend aus 29 Teilen 4-Hydroxymethylpyridinhydrochlorid und 50 Teilen Dihydropyran in 250 Teilen Dimethylformamid wurde 10 Minuten lang trockenes HCl-Gas geleitet. Man ließ die Mischung 5 Tage lang stehen und entfernte dann das Lösungsmittel im Vakuum. Der Rückstand wurde in Essigester aufgenommen und mit einer 10% igen wäßrigen Sodalösung und anschließend mit Wasser gewaschen. Nach dem Trocknen wurde das Lösungsmittel abgezogen und man erhielt 34,5 Teile des Ethers (1). Ausbeute : 89% der Theorie.

b) Herstellung von N-Amino(4-tetrahydropyran-2-yloxy)methyl-pyridinium-mesitylsulfonat (2)

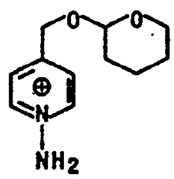 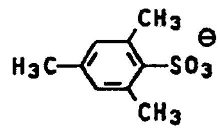

Die Herstellung von (2) aus (1) erfolgte wie in Beispiel 1a angegeben. Es wurde eine Ausbeute von 83% der Theorie erreicht.

c) Herstellung von

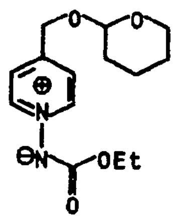 (3)

Analog Beispiel 1 wurde aus dem Mesitylsulfonat (2) der Tetrahydropyranether (3) in 60% iger Ausbeute hergestellt.

d) Herstellung von

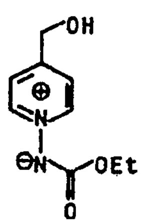 (4)

56 Teile des Tetrahydropyranethers (4) und 5 Teile DOWEX 50W-XZ Ionenaustauscher in 500 Teilen Methanol wurden bei 40 bis 60°C so lange gerührt, bis sich das Ausgangsprodukt nicht mehr dünnschichtchromatographisch nachweisen ließ. Im Anschluß daran wurde der Ionenaustauscher abfiltriert und der Überschuß an Methanol sowie der gebildete Tetrahydropyran-methylether im Vakuum abgezogen. Der so freigesetzte Alkohol (4) wurde ohne weitere Reinigung in der nachfolgenden Reaktion eingesetzt. Ausbeute : 90 % der Theorie.

e) Herstellung von

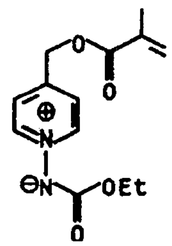

(5)

Zu einer gerührten Mischung aus 39 Teilen des Alkohols (4) und 20 Teilen Triethylamin in 250 Teilen Dichlormethan wurde unter Eiskühlung eine Lösung von 23 Teilen Methacrylchlorid in 100 Teilen Dichlormethan zugetropft. Nach 3stündigem Rühren bei Raumtemperatur wurde das Triethylaminhydrochlorid abgesaugt und die organische Phase mit einer Natriumhydrogencarbonatlösung neutral gewaschen. Nach Abziehen des Lösungsmittels erhielt man 43 Teile des entsprechenden Acrylates (5). Ausbeute : 81 % der Theorie.

<u>Beispiel 7</u>

Wie in Beispielen 6a bis d beschrieben, wurde der Alkohol (4) hergestellt und analog Beispiel 6e hieraus in 62 %iger Ausbeute die folgende Verbindung hergestellt :

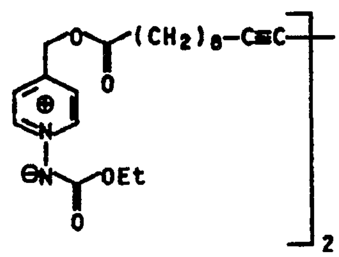

**Ansprüche**

1. Niedermolekulare, photoreaktive Verbindungen der allgemeinen Formeln (I) oder (II)

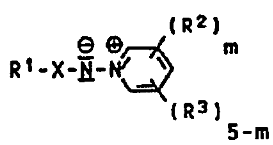

(I)

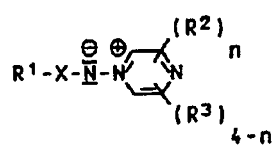

(II)

wobei bedeuten

X: 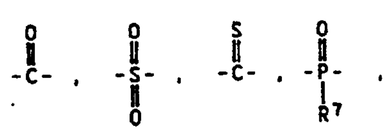

$R^1$ : ein Phenylrest, ein durch Halogen, Alkyl-, Alkoxy-, Amino- oder (Meth)acryloylreste tragende Gruppen substituierter Phenylrest, ein Styrylrest, ein Alkylrest, ein substituierter Alkenylrest, ein vinylisch ungesättigter aliphatischer Rest sowie für den Fall

X= 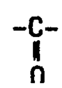 oder 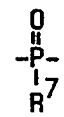

auch eine $-OR^4$-Gruppe oder eine $-NR^5R^6$-Gruppe mit den weiter unter für $R^4$, $R^5$, $R^6$ und $R^7$ angegebenen Bedeutungen ;

$R^2$ : ein Rest mit einer Vinyl-Gruppe, einer Alkenyl-Gruppe, einer Alkinyl-Gruppe, einer Alk-dien-yl-Gruppe, einer Alk-diin-yl-Gruppe, einer Acryloyl-Gruppe, einer Methacryloyl-Gruppe oder einer Fumarsäureoder Maleinsäure-Gruppe ;

$R^3$ : ein Wasserstoffatom, eine,gegebenenfalls substituierte, Alkyl-Gruppe, eine, gegebenenfalls substituierte, Phenyl-Gruppe, ein Halogenatom, eine Amino-Gruppe, ein Alkoxy-Rest, ein phenoxy-Rest, eine Nitro-Gruppe, eine Cyano-Gruppe, ein Alkoxycarbonyl-Rest bzw. ein Aminocarbonyl-Rest oder ein anelliertes alicyclisches, aromatisches oder heteroaromatisches Ringsystem, beispielsweise ein benzoanelliertes Ringsystem ;

$R^4$, $R^6$ : Alkyl, Alkenyl, Alkinyl, Alk-dien-yl, Alk-diin-yl, ein eine Acryloyl-Gruppe oder Methacryloyl-Gruppe enthaltender Rest, Hydroxy- alkyl, ein Oligoalkylenoxid-Rest oder der Styrol-Rest ;

$R^5$ : ein Wasserstoffatom, ein Alkyl-Rest, Cycloalkyl-Rest, der Phenyl-Rest oder ein substituierter Alkyl- oder Phenyl-Rest ;

$R^7$ : ein, gegebenenfalls substituierter, Alkyl-, Alkoxy- oder Aryl-Rest, ein Halogenatom oder eine Hydroxyl-Gruppe ;

m, n : eine ganze Zahl von 1 bis 3.

2. Photoreaktive Verbindung nach Anspruch 1, dadurch gekennzeichnet, daß in den Formeln (I) bzw. (II) $R^2$ einen Rest mit einer oder mehreren aliphatischen Doppelbindungen darstellt.

3. Photoreaktive Verbindungen nach Anspruch 1, dadurch gekennzeichnet, daß in den allgemeinen Formeln (I) bzw, (II) $R^2$ ein Rest mit einer oder mehreren acetylenischen Dreifachbindungen ist.

4. Photoreaktive Verbindungen nach einem der Ansprüche 1 bis 3, dadurch gekennzeichnet, daß in den allgemeinen Formeln (I) bzw. (II) der Rest $R^2$ mehrere, gegebenenfalls miteinander in Konjugation stehende, aliphatische Mehrfachbindungen aufweist.

5. Photoreaktive Verbindungen nach einem der Ansprüche 1 bis 4, dadurch gekennzeichnet, daß in den Formeln (I) bzw. (II) die aliphatischen Mehrfachbindungen in dem Rest $R^2$ in Konjugation zu den Mehrfachbindungen des Pyridinium- bzw. Pyrazinium-Ringes stehen.

6. Photoreaktive Verbindungen nach einem der Ansprüche 1 bis 5, dadurch gekennzeichnet, daß die Verbindungen der allgemeinen Formeln (I) und (II) zwei oder mehr pyridinium-ylid- bzw. Pyrazinium-ylid-Gruppierungen enthalten.

7. Photoreaktive Verbindungen nach Anspruch 6, dadurch gekennzeichnet, daß $R^1$ ein aliphatischer oder aromatischer Rest, vorzugsweise ein Alkyl-, Alkenyl-oder Phenyl-Rest ist, der durch eine Gruppe der Formel (III) oder (IV)

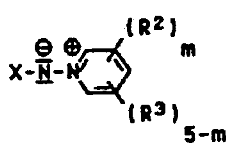

(III)

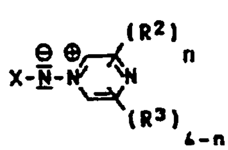

(IV)

8. Photoreaktive Verbindungen nach Anspruch 6, dadurch gekennzeichnet, daß bei den Verbindungen

der allgemeinen Formeln (I) und (II) der Rest $R^3$ eine weitere Iminopyridinium- oder Iminopyrazinium-ylid-Gruppierung enthält.

9. Photoreaktive Verbindungen nach Anspruch 8, dadurch gekennzeichnet, daß $R^3$ ein aliphatischer oder aromatischer Rest oder ein anelliertes Ringsystem ist, welcher bzw. welches eine Gruppierung der allgemeinen Formeln (V) oder (VI)

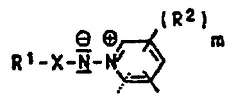 (V)

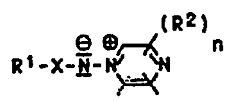 (VI)

enthält, worin $R^1$, $R^2$, X, m und n die oben angegebene Bedeutung haben.

10. Photoreaktive Verbindungen nach einem der Ansprüche 1 bis 7, dadurch gekennzeichnet, daß $R^1$ in den allgemeinen Formeln (I) und (II) sind : Phenyl, p-Methylphenyl, p-Chlorphenyl, o-Chlorphenyl. p-Bromphenyl, p-Aminophenyl, p-Diphenyl, p-Hydroxyphenyl, p-Methoxyphenyl,

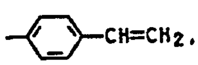, 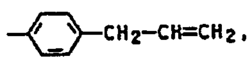, 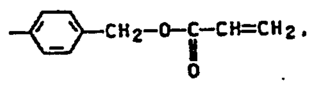,

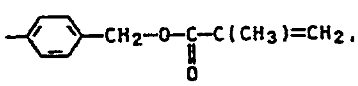, 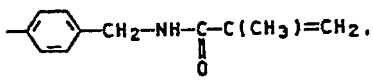,

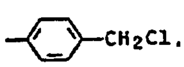, 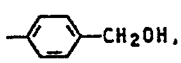, 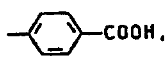, 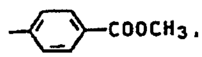,

Methyl, Ethyl, Trifluormethyl, 2-Aminoethyl, $-(CH_2)_4-SH$,

$-CH=CH_2$ oder $-CH_2-CH=CH_2$, aber auch 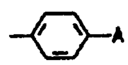, $-CH=CHA$

oder $-(CH_2)_4-A$

mit A gleich einem Rest der vorstehend angegebenen Formel (III) oder (IV) ; oder für den Fall, daß X in den allgemeinen Formeln (I) oder (II) der

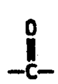 oder 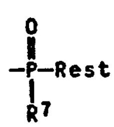

ist, $R^1$ auch eine $OR^4$- oder eine $NR^5R^6$-Gruppe ist.

11. Photoreaktive Verbindungen nach Anspruch 1, dadurch gekennzeichnet, daß der Rest $R^2$ in den

allgemeinen Formeln (I) bzw. (II)

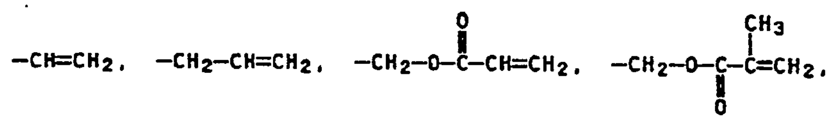

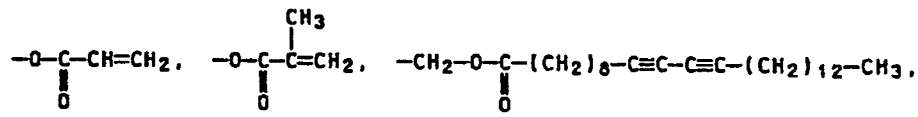

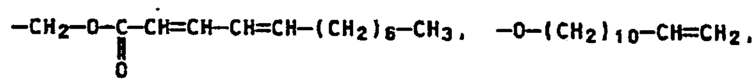

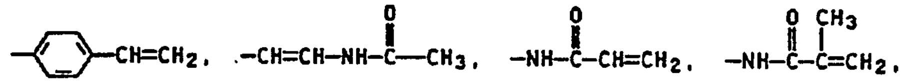

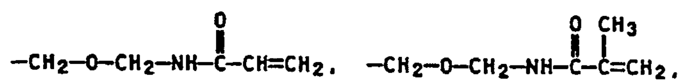

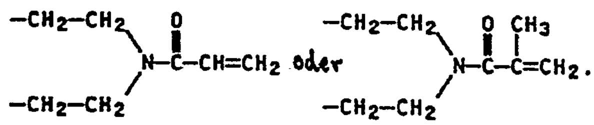

oder

ist.

12. Photoreaktive Verbindungen nach Anspruch 1 oder 9, dadurch gekennzeichnet, daß der $R^3$ in den allgemeinen Formeln (I) bzw. (II) Wasserstoff, Methyl, n-Butyl, Phenyl, Methoxy, Phenoxy,

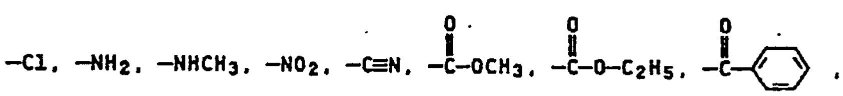

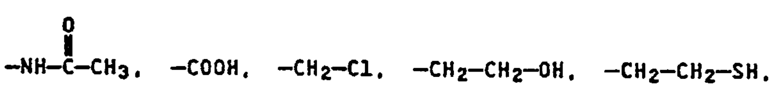

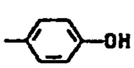 oder 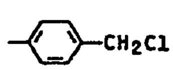

oder ringbildende Reste bzw. anellierte Systeme wie

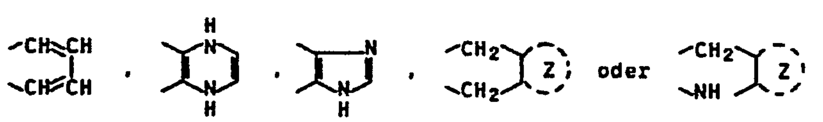

oder

sind, worin Z für ein aromatisches oder heteroaromatisches Ringsystem steht, welches auch eine Gruppierung der vorstehend angegebenen allgemeinen Formeln (V) oder (VI) enthalten kann, oder $R^3$ $-(CH_3)_p-B$ ist, wobei p eine Zahl von 1 bis 10 und B eine Gruppierung der allgemeinen Formel (V) oder (VI) bedeuten.

13. Photoreaktive Verbindungen nach einem der Ansprüche 1 bis 12, dadurch gekennzeichnet, daß die Reste $R^4$ und $R^6$ Methyl, Ethyl, Propyl, Butyl, Phenyl, Allyl, 2-Hydroxyethyl, Tolyl, Xylyl, Aminophenyl,

Styrol,

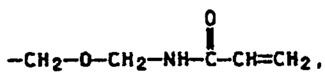, 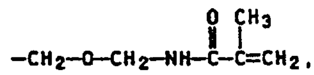,

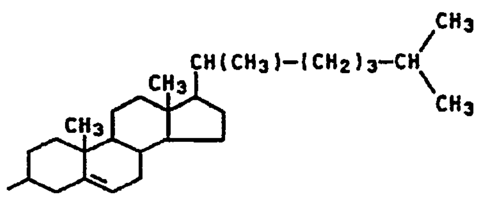 oder

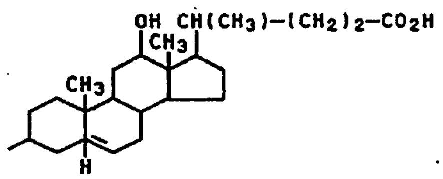

und der Rest $R^5$ Methyl, Ethyl oder Phenyl ist.

14. Photoreaktive Verbindung nach Anspruch 7, dadurch gekennzeichnet, daß der Rest $R^1$ bei den Verbindungen der allgemeinen Formel (I) eine Gruppe der Formel (III) und bei Verbindungen der allgemeinen Formel (II) eine Gruppe der Formel (IV) trägt.

15. Photoreaktive Verbindungen nach Anspruch 9, dadurch gekennzeichnet, daß der Rest $R^3$ bei den Verbindungen der allgemeinen Formel (I) eine Gruppierung der Formel (V) und bei den Verbindungen der Formel (II) eine Gruppierung der Formel (VI) enthält.

## Claims

1. A low molecular weight, photoreactive compound of the formula (I) or (II)

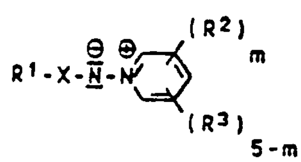 (I)

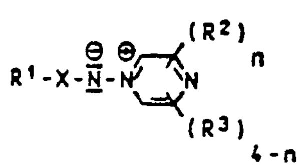 (II)

where

X is 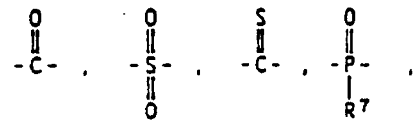

$R^1$ is phenyl which is unsubstituted or substituted by halogen or by groups carrying alkyl, alkoxy, amino or (meth) acryloyl radicals, or is styryl, alkyl, substituted alkenyl, a vinylically unsaturated aliphatic radical and, where

X is 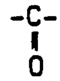 or 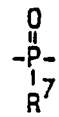 ,

furthermore an $-OR^4$ group or an $-NR^5R^6$ group having the meanings stated further below for $R^4$, $R^5$, $R^6$ and $R^7$ ;

$R^2$ is a radical having a vinyl, alkenyl, alkynyl, alkadienyl, alkadiynyl, acryloyl, methacryloyl or fumaric or maleic acid group ;

$R^3$ is hydrogen, unsubstituted or substituted alkyl, unsubstituted or substituted phenyl, halogen, amino, alkoxy, phenoxy, nitro, cyano, alkoxycarbonyl, aminocarbonyl or a fused alicyclic, aromatic or heteroaromatic ring system, for example a benzofused ring system ; $R^4$ and $R^6$ are each alkyl, alkenyl, alkynyl, alkadienyl, alkadiynyl or a radical containing an acryloyl or methacryloyl group, or are each hydroxyalkyl, an oligoalkylene oxide radical or the styrene radical ;

$R^5$ is hydrogen, alkyl, cycloalkyl, phenyl or a substituted alkyl or phenyl radical ;

$R^7$ is an unsubstituted or substituted alkyl, alkoxy or aryl radical, halogen or hydroxyl, and m and n are each an integer of from 1 to 3.

2. A photoreactive compound as claimed in claim 1, wherein, in the formula (I) or (II), $R^2$ is a radical having one or more aliphatic double bonds.

3. A photoreactive compound as claimed in claim 1, wherein, in the formula (I) or (II), $R^2$ is a radical having one or more acetylenic triple bonds.

4. A photoreactive compound as claimed in claim 1 or 2 or 3, wherein, in the formula (I) or (II), $R^2$ has a plurality of aliphatic multiple bonds which may be conjugated to one another.

5. A photoreactive compound as claimed in claim 1 or 2 or 3 or 4, wherein, in the formula (I) or (II), the aliphatic multiple bonds in $R^2$ are conjugated to the multiple bonds of the pyridinium or pyrazinium ring.

6. A photoreactive compound as claimed in claim 1 or 2 or 3 or 4 or 5, wherein the compounds of the formulae (I) and (II) contain two or more pyridinium-ylide or pyrazinium-ylide groups.

7. A photoreactive compound as claimed in claim 6, wherein $R^1$ is an aliphatic or aromatic radical, preferably alkyl, alkenyl or phenyl, which is substituted by a group of the formula (III) or (IV)

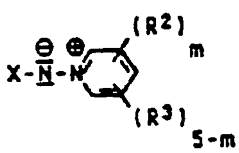

(III)

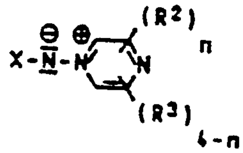

(IV)

where $R^2$, $R^3$, X, m and n have the abovementioned meanings.

8. A photoreactive compound as claimed in claim 6, wherein, in the compounds of the formulae (I) and (II), $R^3$ contains a further iminopyridinium- or iminopyraziniumylide group.

9. A photoreactive compound as claimed in claim 8, wherein $R^3$ is an aliphatic or aromatic radical or a

fused ring system which contains a group of the formula (V) or (VI)

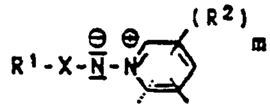 (V)

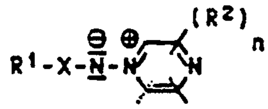 (VI)

where $R^1$, $R^2$, X, m and n have the abovementioned meanings.

10. A photoreactive compound as claimed in claim 1 or 2 or 3 or 4 or 5 or 6 or 7, wherein $R^1$ in the formulae (I) and (II) are phenyl, p-methylphenyl, p-chlorophenyl, o-chlorophenyl, p-bromophenyl, p-aminophenyl, p-biphenyl, p-hydroxyphenyl, p-methoxyphenyl,

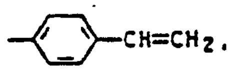, 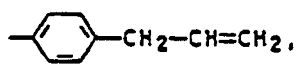, 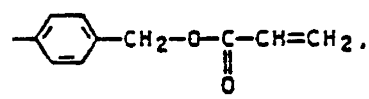,

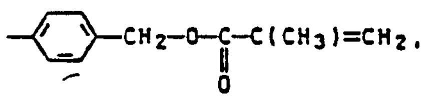, 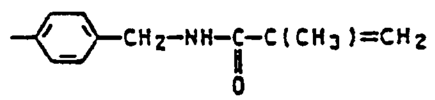,

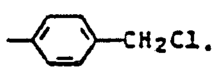, 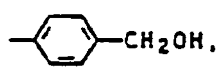, 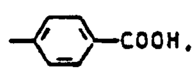, 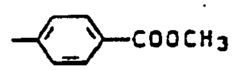,

methyl, ethyl, trifluoromethyl, 2-aminoethyl, $-(CH_2)_4-SH$,

$-CH=CH_2$ or $-CH_2-CH=CH_2$ or 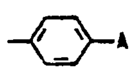 , $-CH=CHA$ or $-(CH_2)_4-A$

where A is a radical of the abovementioned formula (III) or (IV) or, where X in the formula (I) or (II) is

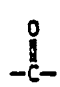 or 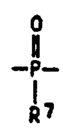 radical

$R^1$ is furthermore an $OR^4$ or an $NR^5R^6$ group.

11. A photoreactive compound as claimed in claim 1, wherein $R^2$ in the formula (I) or (II) is

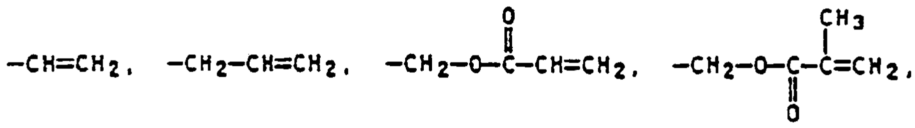

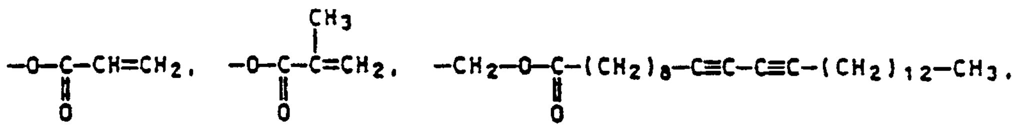

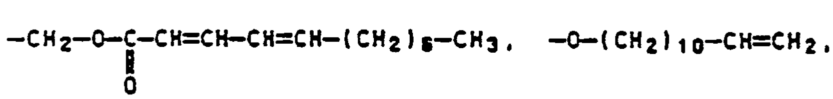

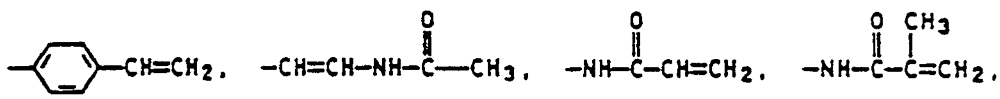

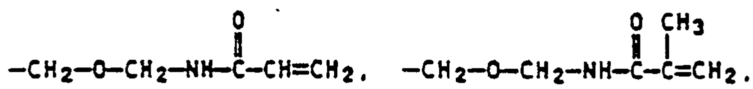

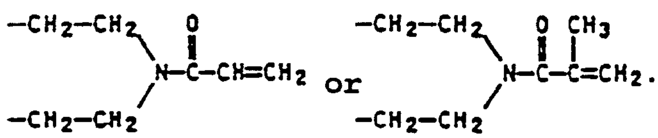

12. A photoreactive compound as claimed in claim 1 or 9, wherein $R^3$ in the formula (I) or (II) is hydrogen, methyl, n-butyl, phenyl, methoxy, phenoxy,

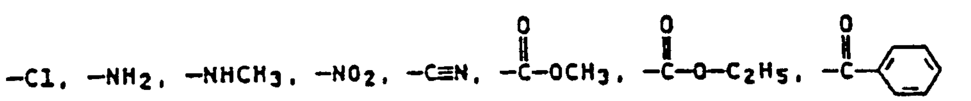

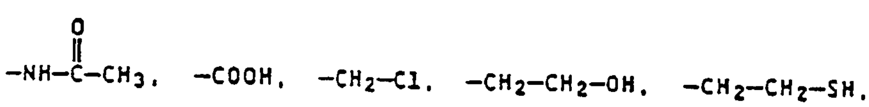

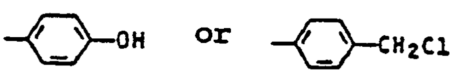

or a ring-forming radical or a fused system, such as

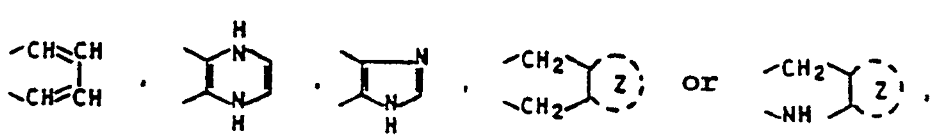

where Z is an aromatic or heteroaromatic ring system which may furthermore contain a group of the abovementioned formula (V) or (VI), or $R^3$ is - $(CH_3)_p$-B, in which p is from 1 to 10 and B is a group of the formula (V) or (VI).

13. A photoreactive compound as claimed in claim 1 or 2 or 3 or 4 or 5 or 6 or 7 or 8 or 9 or 10 or 11 or 12, wherein $R^4$ and $R^6$ are each methyl, ethyl, propyl, butyl, phenyl, allyl, 2-hydroxyethyl, tolyl, xylyl, aminophenyl, styrene

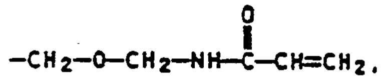 , 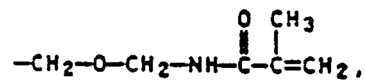 ,

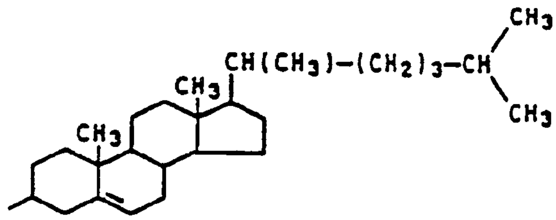 or

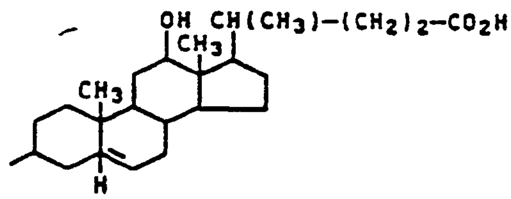

and $R^5$ is methyl, ethyl or phenyl.

14. A photoreactive compound as claimed in claim 7, wherein $R^1$ carries a group of the formula (III) in the compounds of the formula (I) and carries a group of the formula (IV) in compounds of the formula (II).

15. A photoreactive compound as claimed in claim 9, wherein $R^3$ contains a group of the formula (V) in the compounds of the formula (I) and a group of the formula (VI) in the compounds of the formula (II).

## Revendications

1. Composés photoréactifs, de faible poids moléculaire et répondant à la formule générale (I) ou (II)

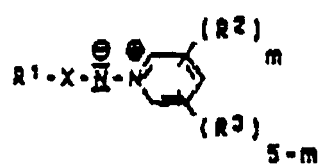 (I)

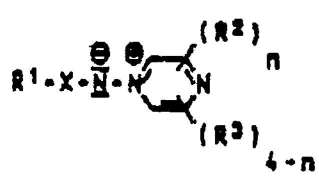 (II)

dans lesquelles X représente

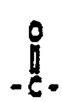 , 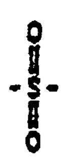 , 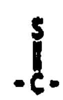 , 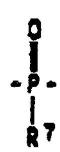 ,

$R^1$ représente un radical phényle, un radical phényle substitué par de l'halogène ou des groupes portant des radicaux alkyle, alcoxy, amino ou (méth)acryloyle, un radical styryle, un radical alkyle, un radical alcényle substitué, un radical aliphatique à insaturation vinylique ainsi que, dans le cas où

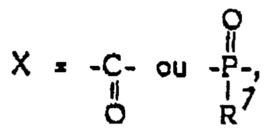

un groupe $-OR^4$ ou un groupe $-NR^5R^6$, avec les significations indiquées ci-dessous pour $R^4$, $R^5$, $R^6$ et $R^7$,

$R^2$ représente un radical comportant un groupe vinyle, alcényle, alcynyle, alc-dién-yle, alc-diyn-yle, acryloyle, méthacryloyle ou un groupe d'acide fumarique ou maléique,

$R^3$ représente un atome d'hydrogène, un groupe alkyle, éventuellement substitué, un groupe phényle, éventuellement substitué, un atome d'halogène, un groupe amino, un radical alcoxy, un radical phénoxy, un groupe nitro, un groupe cyano, un radical alcoxycarbonyle ou respectivement un radical aminocarbonyle, ou un système cyclique condensé alicyclique, aromatique ou hétéroaromatique, par exemple un système cyclique benzocondensé,

$R^4$, $R^6$ représentent un groupe alkyle alcényle, alcynyle alc-dién-yle, alc-diyn-yle, un radical contenant un groupe acryloyle ou méthacryloyle, un groupe hydroxyalkyle, un radical d'oxyde d'oligoalkylène ou le radical styrène,

$R^5$ représente un atome d'hydrogène, un radical alkyle, un radical cycloalkyle, le radical phényle ou un radical alkyle ou phényle substitué,

R 7 représente un radical alkyle, alcoxy ou aryle, éventuellement substitué, un atome d'halogène ou un groupe hydroxyle, m, n représentent un nombre entier de 1 à 3.

2. Composé photoréactif suivant la revendication 1, caractérisé en ce que, dans les formules (I) et respectivement (II), $R^2$ représente un radical comportant une ou plusieurs doubles liaisons aliphatiques.

3. Composés photoréactifs suivant la revendication 1, caractérisés en ce que, dans les formules générales (I) et respectivement (II), $R^2$ représente un radical comportant une ou plusieurs triples liaisons acétyléniques.

4. Composés photoréactifs suivant l'une des revendications 1 à 3, caractérisés en ce que, dans les formules générales (I) et respectivement (II), le radical $R^2$, présente plusieurs liaisons multiples aliphatiques, éventuellement mutuellement disposées en conjugaison.

5. Composés photoréactifs suivant l'une des revendications 1 à 4, caractérisés en ce que, dans les formules (I) et respectivement (II), les liaisons multiples aliphatiques dans le radical $R^2$ sont conjuguées aux liaisons multiples du noyau pyridinium ou pyrazinium.

6. Composés photoréactifs suivant l'une des revendications 1 à 5, caractérisés en ce que les composés des formules générales (I) et (II) contiennent deux ou plusieurs groupements pyridinium-ylide ou respectivement pyrazinium-ylide.

7. Composés photoréactifs suivant la revendication 6, caractérisés en ce que $R^1$ est un radical aliphatique ou aromatique, de préférence un radical alkyle, alcényle ou phényle, qui est substitué par un groupe de la formule (III) ou (IV)

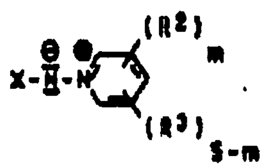

(III)

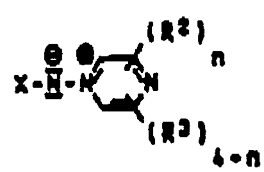

(IV)

dans lesquelles $R^2$, $R^3$, X, m et n ont les significations indiquées précédemment.

8. Composés photoréactifs suivant la revendication 6, caractérisés en ce que, dans les composés des

formules générales (I) et (II), le radical $R^3$ contient un autre groupement iminopyridiniumylide ou iminopyrazinium-ylide.

9. Composés photoréactifs suivant la revendication 8, caractérisés en ce que $R^3$ est un radical aliphatique ou aromatique ou un système cyclique condensé, qui comporte un groupement répondant aux formules générales (V) ou (VI)

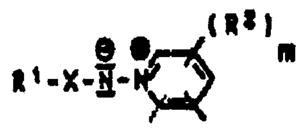 (V)

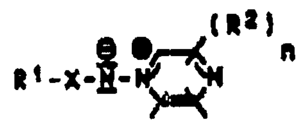 (VI)

dans lesquelles $R^1$, $R^2$ X m et n ont la signification indiquée précédemment.

10. Composés photoréactifs suivant l'une des revendications 1 à 7, caractérisés en ce que, dans les formules générales (I) et (II), $R^1$ est du phényle, du p-méthylphényle, du p-chlorophényle, du o-chlorophényle, du p-bromophényle, du p-aminophényle, du p-diphényle, du p-hydroxyphényle, du p-méthoxyphényle,

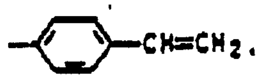, 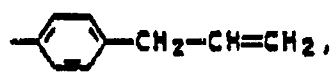, 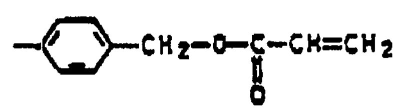,

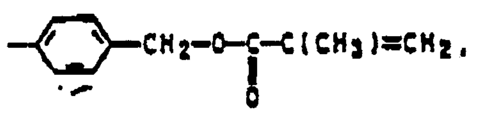, 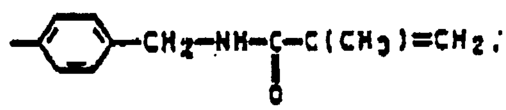;

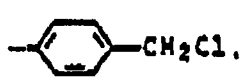, 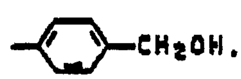, 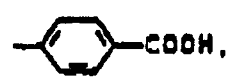, 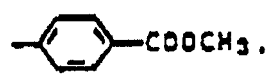,

du méthyle, de l'éthyle, du trifluorométhyle, du 2-aminoéthyle,

$-(CH_2)_4-SH$, $-CH=CH_2$ ou $-CH_2-CH=CH_2$, mais aussi 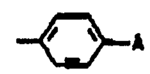,
$-CH=CHA$ ou $-(CH_2)_4-A$

où A est identique à un radical de la formule (III) ou (IV) indiquée précédemment, ou dans le cas où X dans les formules générales (I) ou (II) représente le radical

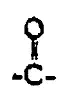 ou 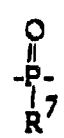

$R^1$ représente également un groupe $OR^4$- ou $NR^5R^6$-.

11. Composés photoréactifs suivant la revendication 1, caractérisés en ce que, dans les formules générales (I) et respectivement (II), le radical $R^2$ représente

$-CH=CH_2$, $-CH_2-CH=CH_2$, 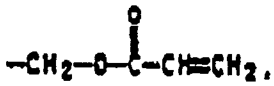, 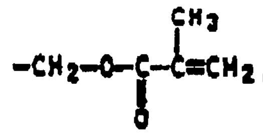,

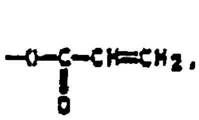, 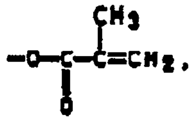, 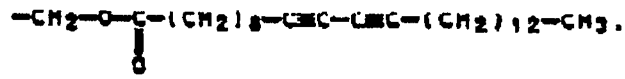,

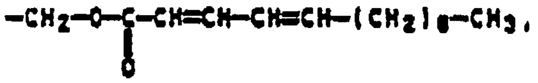, $-O-(CH_2)_{10}-CH=CH_2$,

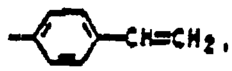, 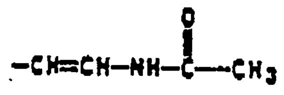, 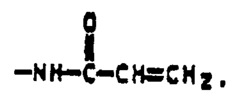, 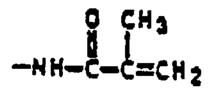,

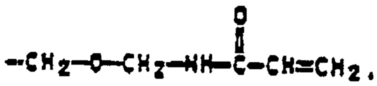, 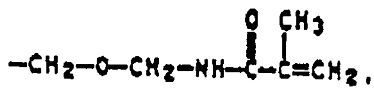,

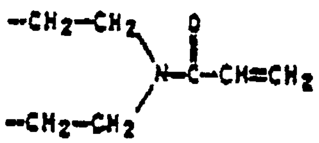 ou 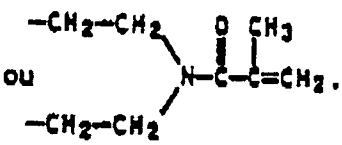.

12. Composés photoréactifs suivant l'une des revendications 1 et 9, caractérisés en ce que, dans les formules générales (I) et respectivement (II), $R^3$ représente de l'hydrogène, du méthyle, du n-butyle, du phényle, du méthoxy, du phénoxy,

$-Cl$, $-NH_2$, $-NHCH_3$, $-NO_2$, $-C\equiv N$, 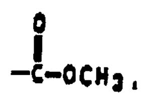, 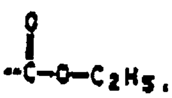, 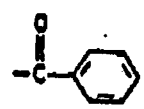,

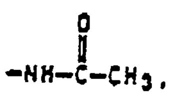, $-COOH$, $-CH_2-Cl$, $-CH_2-CH_2-OH$, $-CH_2-CH_2-SH$,

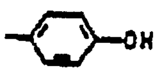 ou 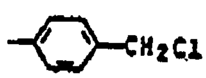

ou des radicaux cyclisants ou respectivement des systèmes condensés, comme

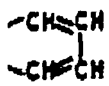, 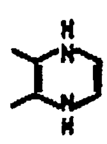, 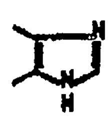, 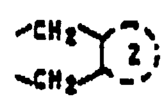 ou 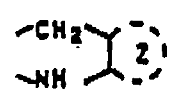,

où Z représente un système cyclique aromatique ou hétéroaromatique, qui peut comporter également un groupement des formules générales (V) ou (VI) indiquées précédemment ou $R^3$ représente $-(CH_3)_p-B$, où p est un nombre de 1 à 10 et D représente un groupement de la formule générale (V) ou (VI).

13. Composés photoréactifs suivant l'une des revendications 1 à 12, caractérisés en ce que les radicaux $R^4$ et $R^6$ représentent du méthyle, de l'éthyle, du propyle, du butyle du phényle, de l'allyle, du 2-hydroxyéthyle, du tolyle, du xylyle, de l'aminophényle, du styrène,

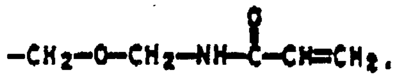 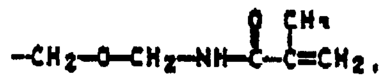

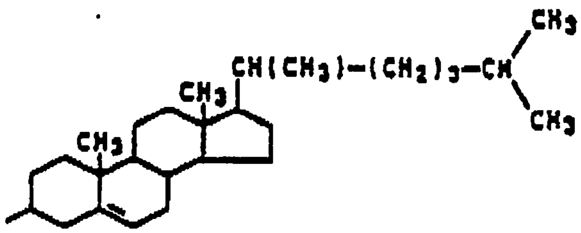

ou

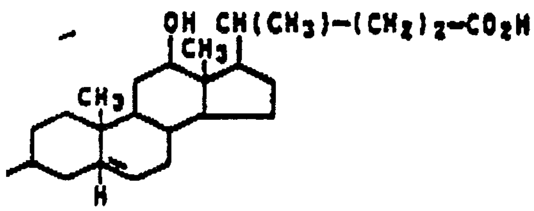

et en ce que le radical $R^5$ représente du méthyle, de l'éthyle ou du phényle.

14. Composé photoréactif suivant la revendication 7, caractérisé en ce que le radical $R^1$ dans les composés de la formule générale (I) porte un groupe de la formule (III) et dans les composés de la formule générale (II) un groupe de la formule (IV).

15. Composés photoréactifs suivant la revendication 9, caractérisés en ce que le radical $R^3$ comporte dans les composés de la formule générale (I) un groupement de la formule (V) et dans les composés de la formule (II) un groupement de la formule (VI).